# EUROPÄISCHE PATENTSCHRIFT

(11) **EP 2 083 255 B1**
(45) Veröffentlichungstag und Bekanntmachung des Hinweises auf die Patenterteilung: **25.01.2012**
(21) Anmeldenummer: 08001466.5
(22) Anmeldetag: 26.01.2008
(51) Int. Cl.: G01N 1/22, G01F 13/00, A61M 16/20

(54) **Gasentnahmevorrichtung**
Gas extraction device
Dispositif d'extraction de gaz

(43) Veröffentlichungstag der Anmeldung: 29.07.2009
(73) Patentinhaber: TESCOM EUROPE GMBH & CO. KG, 23923 Selmsdorf (DE)
(72) Erfinder: Basler. Christian, 23556 Lübeck (DE)
(74) Vertreter: Hemmer, Arnd

(56) Entgegenhaltungen:
- EP-A- 1 475 119
- WO-A-2006/133174
- WO-A-2007/011678
- US-A- 5 785 050
- US-A1- 2005 192 538

## Beschreibung

Die Erfindung betrifft eine Gasentnahmevorrichtung.

Es sind Gasentnahmevorrichtungen beispielsweise in Krankenhäusern oder Laboren bekannt, an welche verschiedenste Geräte und Apparate zu deren Gasversorgung angeschlossen werden können. An der anderen Seite steht die Gasentnahmevorrichtung beispielsweise in Verbindung mit einer zentralen Gasversorgung. An derartigen Gasentnahmevorrichtungen sind häufig verschiedenste Elemente, wie Druckregler, Manometer, Absperrventil und Dosierventil erforderlich, um die Gaszufuhr abzusperren und hinsichtlich Druck und Durchfluss regeln zu können. Die entsprechenden Elemente werden dazu in der gewünschten Weise hintereinander geschaltet und miteinander verbunden, beispielsweise verschraubt. Nachteilig ist dabei zum einen ein großer Aufbau und zum anderen eine entsprechend aufwändige Montage und die Gefahr von Undichtigkeiten an den Verbindungsstellen der einzelnen Elemente.

WO 2007/011678 A1 offenbart einen Verteiler für ein Sauerstoffversorgungssystem. In dem blockförmigen Verteiler sind ein Druckregler sowie ein Drucksensor angeordnet. Weitere Komponenten sind an Anschlussöffnungen des Verteilers angeflanscht. Dies ist insbesondere ein Proportionalventil, welches an eine Öffnung des Verteilers seitlich angesetzt ist. Ferner ist ein Filter an eine Öffnung eines Verteilers angesetzt. Der Aufbau aus einzelnen an den Verteiler angesetzten Komponenten führt zu einem relativ großen und in der Gestaltung nicht besonders ansprechenden Aufbau des Sauerstoffversorgungssystems.

US 5,785,050 offenbart ein Sauerstoffventilsystem, welches vorgesehen ist, um direkt auf eine Sauerstoffflasche aufgeschraubt zu werden. Das Ventilsystem enthält ein über eine Membran betätigtes Ventil, welches durch die Atmung eines Patienten gesteuert wird und abhängig von der Atmung den Strömungsweg für den Sauerstoff freigibt oder verschließt. Darüber hinaus ist eine Druckreduziereinrichtung in das Ventilsystem integriert. Ausgangsseitig ist auch noch ein Durchflussmesser vorgesehen. Die Druckreduziereinrichtung ist jedoch nicht einstellbar. Darüber hinaus ist auch kein Dosierventil vorgesehen. Daher eignet sich diese Vorrichtung nicht als herkömmliche Gasentnahmevorrichtung, wie sie beispielsweise in Laboren eingesetzt wird.

EP 1 475 119 A1 offenbart eine Vorrichtung zur Steuerung einer Gasströmung, wie sie beispielsweise in Beatmungsgeräten eingesetzt werden kann. Die Vorrichtung enthält eine Verstärkerventil- und eine Dosiereinrichtung, welche in einem Ventilblock angeordnet sind. Auch diese Vorrichtung eignet sich nicht als klassische Gasentnahmevorrichtung, hier fehlt als wesentliche Komponente ein Druckregler.

US 2005/0192538 A1 offenbart eine Vorrichtung zur Regelung einer FluidStrömung, welche ebenfalls für die Beatmung von Patienten vorgesehen ist.

Es ist Aufgabe der Erfindung, eine verbesserte Gasentnahmevorrichtung zu schaffen, welche kompakt aufgebaut und einfach zu montieren ist sowie die Gefahr von Undichtigkeiten zwischen einzelnen Bestandteilen der Gasentnahmevorrichtung verringert.

Diese Aufgabe wird durch eine Gasentnahmevorrichtung mit den in Anspruch 1 angegebenen Merkmalen gelöst. Bevorzugte Ausführungsformen ergeben sich aus den Unteransprüchen, der nachfolgenden Beschreibung sowie den beigefügten Figuren.

Die erfindungsgemäße Gasentnahmevorrichtung weist als Minimalkombination einen Druckregler sowie ein Dosierventil auf. Der Druckregler dient zur Einstellung des Drucks des zu entnehmenden Gases und das Dosierventil zur Einstellung des Durchflusses. Beide Elemente sind im Strömungsweg in Reihe geschaltet, wobei das Dosierventil vorzugsweise stromabwärts des Druckreglers, d. h. an der Ausgangsseite des Druckreglers angeordnet ist. Erfindungsgemäß sind der Druckregler und das Dosierventil in einen gemeinsamen Ventilblock integriert. D. h. Druckregler und Dosierventil bilden erfindungsgemäß ein integriertes Bauteil. Dies hat den Vorteil, dass zum einen ein kompakter Aufbau erreicht werden kann und zum anderen es nicht erforderlich ist, zwei Einzelteile nämlich Druckregler und Dosierventil zur Ausbildung der Gasentnahmevorrichtung miteinander zu verbinden. Auf diese Weise kann eine mögliche Undichtigkeit in der Verbindungsstelle vermieden werden. Der kompakte Aufbau bietet darüber hinaus auch die Möglichkeit das Erscheinungsbild der gesamten Gasentnahmevorrichtung moderner und ansprechender zu gestalten. Ferner kann dadurch ein übersichtlicherer Aufbau erreicht werden, welcher auch die Bedienbarkeit für den Benutzer verbessert. Die Integration von Dosierventil und Druckregler in einen Ventilblock ermöglicht nämlich darüber hinaus eine übersichtliche und leicht zu handhabende Anordnung der jeweiligen Bedienelemente für Druckregler und Dosierventil.

Gemäß einer bevorzugten Ausführungsform ist in den Ventilblock zusätzlich ein Absperrventil integriert, welches dazu dient, die Gaszufuhr vollständig abzusperren. Dies ist insbesondere dann bevorzugt, wenn das Dosierventil nicht zur vollständigen Absperrung der Gaszufuhr ausgebildet ist und stets einen Minimaldurchfluss zulässt. Das Absperrventil ist vorzugsweise in Flussrichtung vor dem Druckregler, d. h. eingangsseitig des Druckreglers angeordnet. Es ist jedoch auch möglich, das Absperrventil ausgangsseitig des Druckreglers beispielsweise im Strömungsweg zwischen Druckregler und Dosierventil, aber auch stromabwärts des Dosierventils anzuordnen. Dabei bildet dann das Absperrventil mit dem Dosierventil und dem Druckregler in dem gemeinsamen Ventilblock ein integriertes Bauteil, welches eine kompakte Ausgestaltung des gesamten Gasentnahmevorrichtung und darüber hinaus eine übersichtliche Anordnung der Bedienelemente ermöglicht. Insbesondere können alle Bedienelemente nahe beieinander angeordnet werden, so dass eine Einhandbedienung ohne aufwändiges Umgreifen der Gasentnahmevorrichtung möglich wird.

Ferner kann vorzugsweise an dem Ventilblock auch ein Manometer angesetzt sein. Ein solches Manometer kann direkt in den Ventilblock integriert sein, so dass eine Anzeigevorrichtung an einer Außenseite bzw. Oberfläche des Ventilblockes gelegen ist. Alternativ kann in dem Ventilblock ein Anschluss ausgebildet sein, an welchen das Manometer angesetzt ist, so dass es insbesondere mit seinem Anzeigeelement aus dem Ventilblock hervorragt. Der Ventilblock kann zusätzlich mit einer Verkleidung oder einem Gehäuse umgeben sein, welches die Gasentnahmevorrichtung nach außen verkleidet. Wenn ein solches Gehäuse vorgesehen ist, kann das Manometer, auch wenn es außen an den Ventilblock angesetzt ist, gemeinsam mit dem Ventilblock in ein gemeinsames, umschließendes Gehäuse integriert sein. Während der Ventilblock vorzugsweise aus Metall gefertigt ist, kann das Gehäuse als Spritzgussteil aus Kunststoff ausgebildet sein, welches eine vielfältige Formgebung erlaubt.

In dem Ventilblock sind Verbindungskanäle zur Verbindung von Druckregler und Dosierventil und vorzugsweise Absperrventil und/oder Manometer ausgebildet. D. h. bevorzugt werden alle erforderlichen Verbindungen bzw. Strömungswege von dem Eingang der Gasentnahmevorrichtung, welcher zum Anschluss beispielsweise an eine zentrale Gasversorgung vorgesehen ist, bis zum Ausgang, an welchen mit Gas zu versorgende Geräte angeschlossen werden können, im Inneren des Ventilblocks ausgebildet. Auf diese Weise entfallen zwischen allen Elementen im Ventilblock, d. h. Druckregler, Dosierventil und gegebenenfalls Manometer und/oder Absperrventil zusätzliche Leitungsverbindungen, welche den Montageaufwand und die Gefahr von Undichtigkeiten vergrößern würde. Alle Verbindungen bzw. Strömungswege können vorzugsweise als Kanäle oder Löcher, welche beispielsweise als Bohrungen gefertigt werden können, im Inneren des Ventilblocks ausgebildet werden.

Besonders bevorzugt ist der Ventilblock einstückig ausgebildet. So kann der Ventilblock aus einem Metallteil gefertigt werden. Die erforderlichen Aufnahmen für den Druckregler, das Dosierventil und/oder das Absperrventil und/oder das Manometer sowie die erforderlichen Verbindungskanäle und Anschlüsse können in ein solches Metallteil insbesondere spanend oder auch auf andere geeignete Weise eingebracht werden. Für den Druckregler und die Ventile, d. h. Absperr- und Dosierventil werden in dem Ventilblock vorzugsweise zur Oberfläche des Ventilblocks hin geöffnete Aufnahmen in Form von Löchern oder Ausnehmungen ausgebildet, in welche die genannten Elemente eingesetzt werden. Die Verbindungskanäle im Inneren des Ventilblockes münden in diese Aufnahmen. In den Aufnahmen können gegebenenfalls auch direkt die erforderlichen Ventilsitze ausgebildet sein. Alternativ können die Ventilsitze als separate Bauteile in die Aufnahmen eingesetzt werden.

Der Druckregler ist an einer ersten Stirnseite des Ventilblocks angeordnet. Dies ist vorzugsweise diejenige Stirnseite, welche in Einbaulage der Gasentnahmevorrichtung nach vorne weist, d. h. einer Bedienperson zugewandt ist. So kann an der Vorderseite der Gasentnahmevorrichtung ein Handrad zur Betätigung des Drucksreglers angeordnet werden. Der Ventilblock ist vorzugsweise im Wesentlichen zylindrisch ausgebildet und die Drehachse des Handrades erstreckt sich entlang der Längsachse des Ventilblocks. Der Ventilblock hat dabei vorzugsweise einen Außendurchmesser, welcher im Wesentlichen dem Außendurchmesser des Handrades des Druckreglers entspricht, so dass ein kompakter Aufbau und ein integrales Erscheinungsbild der Gasentnahmevorrichtung erreicht werden.

Das Dosierventil und vorzugsweise auch das Absperrventil sind an einer Umfangsfläche des Ventilblocks angeordnet. D. h., dass in dem Ventilblock Aufnahmen bzw. Ausnehmungen ausgebildet sind, welche zu der Umfangsfläche hin geöffnet sind und in welche die entsprechenden Ventile eingesetzt sind. Vorzugsweise stehen die Ventile dabei im Wesentlichen nicht über die Umfangsfläche des Ventilblocks vor, sondern allenfalls Betätigungselemente für diese Ventile. Die Umfangsfläche, in der die Ventile, d. h. Dosierventile und gegebenenfalls Absperrventile angeordnet sind, ist vorzugsweise eine Umfangsfläche, welche sich normal zur der Stirnseite erstreckt, an welcher der Druckregler angeordnet ist. Bevorzugt handelt es sich dabei um eine im Wesentlichen zylindrische Umfangsfläche.

Ferner sind an einer zweiten Stirnseite des Ventilblocks und/oder an eine Umfangsfläche zumindest ein Gaseingang und ein Gasausgang angeordnet. Die zweite Stirnseite ist dabei bevorzugt diejenige Stirnseite, welche der ersten Stirnseite, an welcher der Druckregler angeordnet ist, abgewandt ist. In Einbaulage ist dies die rückseitige Stirnseite der Gasentnahmevorrichtung. Es ist möglich, alternative Gasein- und/oder ausgänge an dem Ventilblock auszubilden. So können beispielsweise ein rückseitiger Gasausgang an der Stirnseite und ein umfänglicher Gasausgang an der Umfangsfläche vorgesehen sein, welche alternativ angeschlossen werden können. Der nicht genutzte Gasausgang kann dann durch eine Schraube oder einen Blindstopfen verschlossen sein. Entsprechend kann beispielsweise auch ein Gaseingang an der zweiten Stirnseite und ein umfänglicher Gaseingang vorgesehen sein, welche alternativ genutzt werden können, um unterschiedliche Anschluss- bzw. Einbaulagen mit ein und derselben Gasentnahmevorrichtung bzw. ein und demselben Ventilblock realisieren können. Der nicht genutzte Gaseingang kann dann entsprechend beispielsweise durch eine Dichtschraube bzw. Blindstopfen verschlossen sein. Darüber hinaus ist es möglich, mehrere Gasein- und/oder mehrere Gasausgänge so anzuordnen, dass es optional möglich ist, einzelne Komponenten wie Absperr- oder Dosierventil in dem Ventilblock zu umgehen. So können beispielsweise zwei Gasausgänge vorgesehen sein, von denen einer vor dem Dosierventil und einer in Strömungsrichtung hinter dem Dosierventil angeordnet ist. Dies ermöglicht es, alternativ auch den vollen Durchfluss ohne Wirkung des Dosierventils zusätzlich zu dem durch das Dosierventil verringerten Durchfluss aus der Gansentnahmevorrichtung zu entnehmen.

Zur Betätigung des Dosierventils ist vorzugsweise ein den Ventilblock umfänglich umgebender Drehring angeordnet, welcher um die Längsachse des Ventilblocks drehbar ist. In dem Fall, dass der Ventilblock von einem umgebenden Gehäuse umhüllt ist, ist dieser Drehring vorzugsweise in die Oberfläche des Gehäuses eingesetzt oder umgibt die Gehäuseoberfläche umfänglich. Ein solcher Drehring, welcher den gesamten Ventilblock bzw. die gesamte Gasentnahmevorrichtung umfänglich umgibt, kann sehr gut ergriffen werden. Ferner ermöglicht ein derart großer Drehring eine genaue Dosierung bei vergleichsweise kleinem Drehwinkel, da ein großer Drehring auch über einen kleinen Drehwinkel sehr feinfühlig eingestellt werden kann. So ist es möglich, eine Ausgestaltung zu realisieren, bei welcher der Stellweg für den Drehring zur Betätigung des Dosierventils weniger als 360 ° und weiter bevorzugt weniger als 180 ° beträgt. So ist ein feinfühliges Dosieren ohne Umgreifen möglich. Der Drehring kann ferner mit Rastungen versehen sein, welche dem Benutzer ein besseres Gefühl beim Einstellen des Dosierventils vermitteln. So können besonders bevorzugt die einzelnen Rastungen festgelegten Durchflussänderungen entsprechen. Die Raststufen sind vorzugsweise sehr klein gewählt, damit eine genaue und feinfühlige Dosierung möglich ist.

Der Drehring ist vorzugsweise mit einer Verzahnung versehen, welche mit einem Zahnrad einer Ventilspindel des Dosierventils kämmt, wobei sich die Längsachse der Ventilspindel vorzugsweise normal zur Längsachse des Ventilblocks erstreckt. Die Ventilspindel ist vorzugsweise gemeinsam mit den übrigen Elementen des Dosierventils, insbesondere einem Ventilsitz in einer Ausnehmung bzw. Bohrung, welche sich radial ausgehend von der Umfangsfläche in das Innere des Ventilblocks erstreckt, eingesetzt. Die Ventilspindel läuft in einem Gewinde, so dass die Drehbewegung der Ventilspindel in eine Hubbewegung zum Öffnen und Schließen des Ventils umgesetzt wird. Die Ventilspindel steht mit einem Ende vorzugsweise geringfügig über den Außenumfang des Ventilblocks vor, so dass dort ein Zahnrad ausgebildet oder angeordnet sein kann. Die Verzahnung des Drehringes dreht bei dessen Drehung das Zahnrad, wodurch die Ventilspindel gedreht wird. Die Drehachse von Drehring und Ventilspindel sind dabei normal zueinander angeordnet. Die Verzahnung an dem Drehring muss sich nicht über den gesamten Umfang des Drehringes erstrecken, wenn der Verstellweg des Drehringes weniger als 360°. Die Verzahnung ist vorzugsweise so angeordnet, dass die Zähne der Verzahnung am Drehring sich vorzugsweise parallel zur Umfangsfläche des Ventilblocks, d. h. auf einer Zylindermantelfläche konzentrisch zur Drehachse des Drehringes erstrecken. Die Verzahnung des Zahnrades ist entsprechend als Stirnverzahnung ausgebildet. Alternativ wäre auch eine Paarung nach Art einer Kegelradverzahnung möglich. Insbesondere bei dieser Ausgestaltung könnte dann die Ventilspindel auch so in einem Gewinde gelagert werden, dass sich nicht die Ventilspindel in axialer Richtung bewegt, sondern ein mit der Ventilspindel über deren Gewinde im Eingriff befindliches zweites Element, welches dann das Ventil durch Axialbewegung öffnet und schließt.

Weiter bevorzugt ist der Drehring konzentrisch zu einem Handrad des Druckreglers angeordnet. Dabei sind der Drehring und das Handrad des Druckreglers vorzugsweise um dieselbe Drehachse, d. h. weiter bevorzugt um die Längsachse des Ventilblocks drehbar. So kann eine sehr kompakte Ausgestaltung der gesamten Gasentnahmevorrichtung erreicht werden. Vorzugsweise ist der Drehring zu dem Handrad des Druckreglers in axialer Richtung entlang der Drehachse versetzt angeordnet. Dabei liegt von der ersten Stirnseite aus gesehen der Drehring vorzugsweise axial hinter dem Handrad des Druckreglers bzw. an dessen Axialende. Weiter bevorzugt schließen beide Teile direkt aneinander an oder der Drehring überdeckt das Axialende des Handrades des Druckreglers umfänglich. Auf diese Weise wird eine Außenkontur mit wenigen Fugen und eine insgesamt harmonische Außengestaltung des Druckreglers erzielt.

Zur Betätigung des Absperrventils ist vorzugsweise ein Schiebeelement angeordnet, welches parallel zur Längsachse des Ventilblocks, d. h. vorzugsweise parallel zur Drehachse des Drehringes und/oder eines Handrades eines Druckreglers bewegbar ist. Das Schiebeelement ist dabei vorzugsweise direkt an der Außenfläche des Ventilblocks oder eines den Ventilblock umgebenden Gehäuses angeordnet, so dass es auch harmonisch und vorzugsweise bündig in die übrige Gehäusegestaltung integriert werden kann. Alternativ kann das Schiebeelement auch in Umfangsrichtung verschiebbar sein und insbesondere ebenfalls als Drehring ausgebildet sein.

Besonders bevorzugt ist das Schiebeelement an einem Drehring zur Betätigung des Dosierventils parallel zur Drehachse des Drehringes linear beweglich geführt. D. h. das Schiebeelement kann in die Grundform bzw. die Außenkontur des Drehringes integriert werden, so dass alle Bedienelemente der Gasentnahmevorrichtung leicht ergreifbar und nah beieinander liegen. Dabei kann das Schiebeelement so ausgebildet bzw. in dem Drehring angeordnet sein, dass es gemeinsam mit dem Drehring verdrehbar ist. Es wäre möglich, den gesamten Drehring gleichzeitig als Schiebeelement auszubilden, so dass er neben einer Drehbewegung auch eine Axialbewegung parallel zu seiner Drehachse ausführen kann, um bei dieser Bewegung das Absperrventil zu betätigen. Bevorzugt erstreckt sich das Schiebeelement jedoch nicht über den gesamten Umfang des Drehringes, sondern nur um ein Teilsegment des Umfanges und ist dort axial beweglich in dem Drehring geführt.

Das Absperrventil selber wird bevorzugt über eine Kipphebelmechanik betätigt, welche vorzugsweise über ein Schiebeelement betätigbar ist. Dabei kann das Schiebeelement in der zuvor beschriebenen Weise ausgestaltet bzw. angeordnet sein. Die Kipphebelmechanik dient dabei dazu, eine Axialbewegung parallel zur Längsachse des Ventilblocks oder eine Bewegung in Umfangsrichtung in eine Axialbewegung normal zu der Längsachse umzusetzen. Das Absperrventil selber ist vorzugsweise in einer Ausnehmung bzw. einem Sackloch angeordnet, welches sich ausgehend von der Umfangsfläche des Ventilblockes radial zu dessen Längsachse erstreckt. Die Betätigungsbewegung eines Ventilelementes, beispielsweise eines Kolbens oder einer Membran erfolgt dabei vorzugsweise ebenfalls in radialer Richtung. Ein Kipphebel kann diese Bewegung bewirken, wenn das Schiebeelement außerhalb der Außenkontur des Ventilblockes parallel zu dessen Längsachse oder in dessen Umfangsrichtung verschoben wird und auf den Kipphebel wirkt. Vorzugsweise ist das Absperrventil oder dessen Betätigungselement mit Rastpositionen versehen, welche der geöffneten und der geschlossen Position des Absperrventiles entsprechen. Hierzu können geeignete Rastelemente, beispielsweise Rastkugeln vorgesehen sein. Darüber hinaus ist es möglich die Kipphebelmechanik als Über-Totpunktmechanik auszubilden, wodurch erreicht wird, dass der Kipphebel in seinen zwei End- bzw. Schaltstellungen einrastet.

Gemäß einer weiteren bevorzugten Ausführungsform kann der Drehring zur Betätigung des Dosierventils in axialer Richtung mit einer Federkraft belastet sein, welche die Verzahnung des Drehringes mit der Verzahnung des Zahnrades an der Ventilspindel zusammendrückt. Auf diese Weise kann das Spiel aus dem Getriebeeingriff zwischen Verzahnung und Zahnrad beseitigt werden, so dass eine genaue Verstellung des Dosierventiles über den Drehring möglich ist.

Das Manometer ist vorzugsweise als gestecktes Manometer mit dem Ventilblock verbunden. D. h. hier ist in dem Ventilblock eine Anschlussausnehmung in Form eines Sackloches ausgebildet, in welche das Manometer mit einem Anschlusselement eingesteckt wird. Gegen Herausfallen ist das Manometer dann mittels einer Madenschraube in dem Ventilblock gesichert, wobei die Madenschraube vorzugsweise in einer Ausnehmung oder Nut an dem Anschlusselement des Manometers eingreift. Das Anschlusselement und die Ausnehmung an dem Ventilblock können so ausgebildet sein, dass ein Einsetzen nur in einer bestimmten Winkelposition möglich ist, so dass bei der Montage automatisch eine richtige Ausrichtung von Manometer und Ventilblock zueinander erreicht wird.

Die Anschlüsse, d. h. Gasein- und -ausgänge an dem Ventilblock sind vorzugsweise so ausgebildet, dass sie sowohl mit einem Gewinde als auch mit einem Sitz für einen O-Ring versehen sind. Auf diese Weise sind verschiedene Abdichtungs- oder Anbindungsarten möglich. Nämlich zum einen eine Abdichtung allein über das Gewinde oder zum anderen bei anderweitiger Verbindung, beispielsweise Klemmverbindung, mittels eines O-Ringes.

Nachfolgend wird die Erfindung beispielhaft anhand der beigefügten Figuren beschrieben. In diesen zeigt:
- Fig. 1: eine perspektivische Gesamtansicht einer efindungsge- mäßen Gasentnahmevorrichtung schräg von vorne,

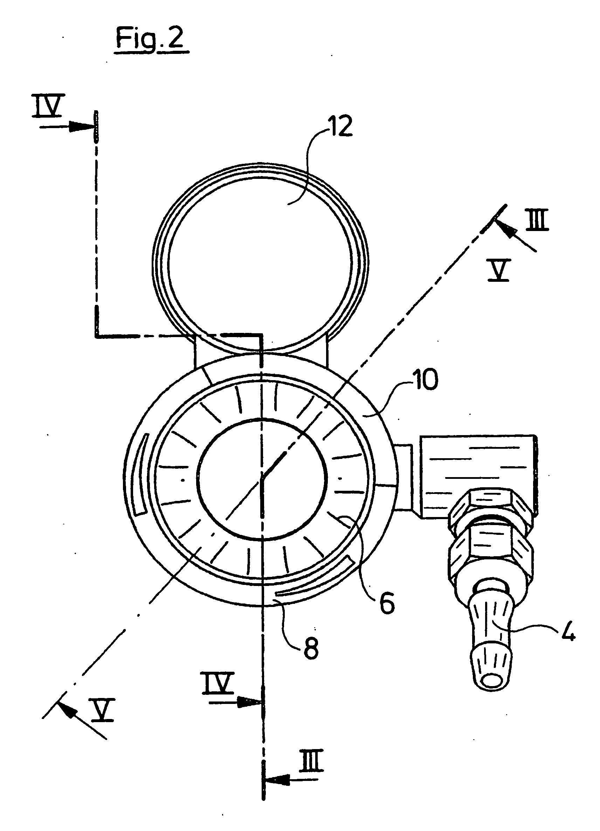

- Fig. 2: eine Frontansicht der Gasentnahmevorrichtung gemäß Fig. 1,
- Fig. 3: eine Schnittansicht entlang Linie III-III in Fig. 2,
- Fig. 4: eine Schnittansicht entlang Linie IV-IV in Fig. 2,
- Fig. 5: eine Schnittansicht entlang der Linie V-V in Fig. 2,

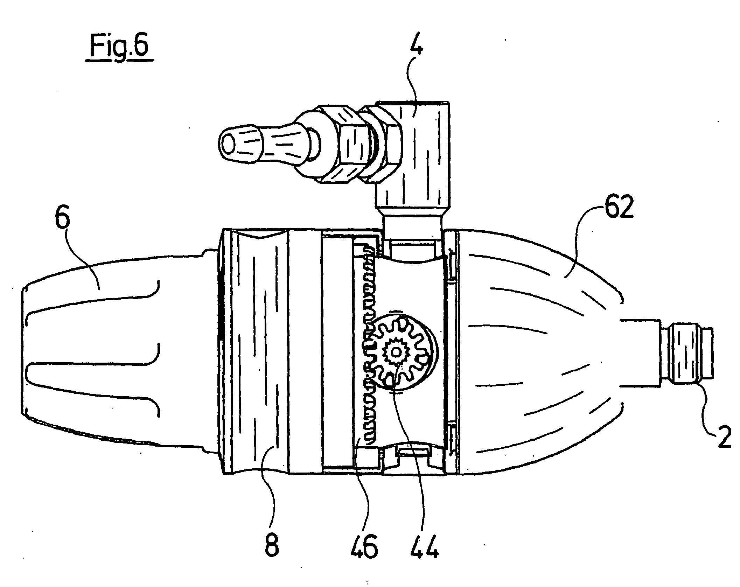

- Fig. 6: eine Seitenansicht der Gasentnahmevorrichtung gemäß der vorangehenden Figuren, wobei ein Teil des Drehringes zur Betätigung des Dosierventiles entfernt ist,
- Fig. 7: eine weitere Seitenansicht der erfindungsgemäßen Gas- entnahmevorrichtung und
- Fig. 8: eine Schnittansicht entlang der Linie VIII-VIII in Fig. 7.

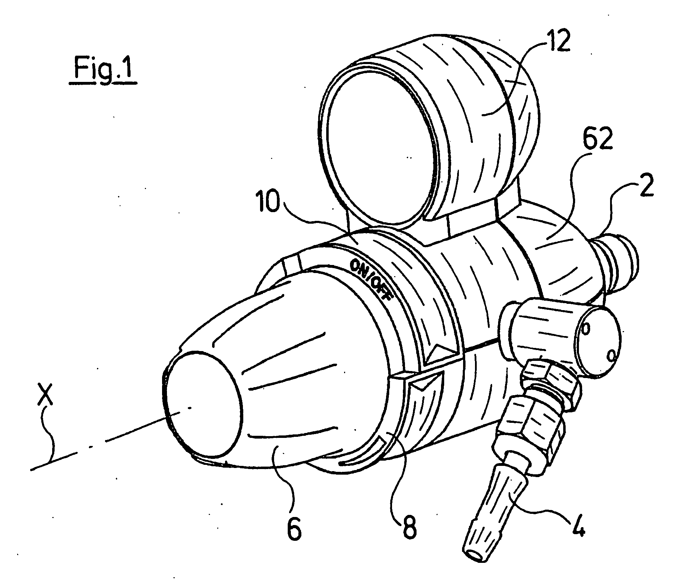

Die gezeigte Gasentnahmevorrichtung dient zur Entnahme von Gas aus einer Gasquelle oder einer Gasversorgungseinrichtung. Zur Verbindung mit einer solchen Gasquelle oder Gasversorgungseinrichtung weist die Gasentnahmevorrichtung einen Anschluss 2 auf, welcher den Gaseingang bildet, und einen Anschluss 4 auf, welcher den Gasausgang bildet. Der Anschluss 4 dient zur Verbindung mit externen Geräten oder Anlagen, welche mit Gas versorgt werden sollen und kann auch abweichend von der hier gezeigten Weise zur Verbindung mit einem Anschlussschlauch ausgebildet sein. Die hier gezeigte Gasentnahmevorrichtung integriert in ein zentrales Bauteil drei wesentliche Elemente, nämlich einen Druckregler, ein Dosierventil und ein Absperrventil, für die drei Betätigungselemente an der Außenseite des Gehäuses ausgebildet bzw. angeordnet sind. Dies ist zunächst ein Handrad 6 zur Betätigung des Druckreglers. Das Handrad 6 ist front- bzw. stirnseitig angeordnet und um die Längsachse X drehbar. Ferner ist ein Drehring 8 vorgesehen, welcher in Richtung der Längsachse X axial versetzt am rückwärtigen Ende des Handrates 6 angeordnet ist und konzentrisch zu diesem um die Längsachse X drehbar ist, um ein Dosierventil im Inneren der Gasentnahmevorrichtung zu betätigen. Ferner ist ein Schiebeelement 10 zur Betätigung eines Absperrventiles vorgesehen, welches parallel zur Längsachse X verschiebbar ist, um das Absperrventil zu öffnen oder zu schließen. In der in Fig. 1 gezeigten axial nach hinten, d. h. von der Frontseite des Handrades 6 verschobenen Positionen, befindet sich das Schiebeelement 10 in der Absperrposition, in welcher das Absperrventil verschlossen ist. In dem hier gezeigten Ausführungsbeispiel ist das Schiebeelement 10 als Segment des Drehringes 8 ausgebildet und in diesem axial verschiebbar geführt. Das Schiebeelement 10 wird gemeinsam mit dem Drehring 8 verdreht, ist aber unabhängig von dessen Drehbewegung verschiebbar. So kann ein Absperren der Gasentnahmevorrichtung ohne Änderung der Dosiereinstellung erreicht werden.

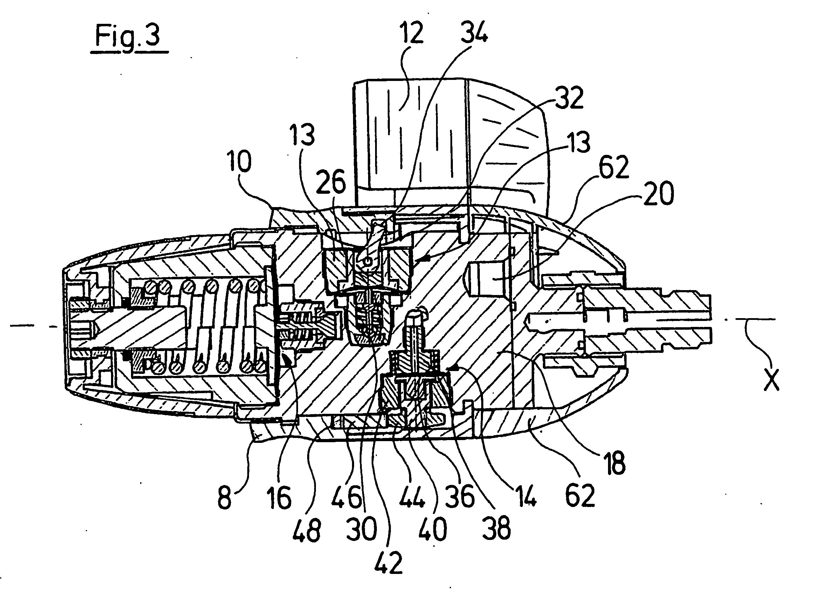

Ferner ist in Fig. 1 zu erkennen, dass an der Außenseite des Gehäuses ein Manometer 12 angeordnet ist. Wie insbesondere in den Fig. 3, 4 und 5 zu erkennen ist, sind die drei wesentlichen Elemente, Absperrventil 13, Dosierventil 14 und Druckregler 16 in einen gemeinsamen Ventilblock 18 integriert. Der Ventilblock 18 ist einstückig aus Metall gefertigt. Der Druckregler 16, das Dosierventil 14 und das Absperrventil 13 sind in Ausnehmungen bzw. Löcher in dem Ventilblock 18 eingesetzt. Dabei ist der Druckregler 16 an einer ersten vorderen Stirnseite des Ventilblockes 18 angeordnet. Die Aufnahmen für das Absperrventil 13 und das Dosierventil 14 erstrecken sich im Wesentlichen in radialer Richtung zu der Längsachse X des Ventilblockes 18 von der Umfangsfläche her. Der Ventilblock 18 ist dabei im Wesentlichen zylindrisch ausgebildet.

Der Strömungsweg im Inneren des Ventilblockes 18 ist durch Bohrungen oder Kanäle gebildet, welche die einzelnen Elemente miteinander verbinden, in den hier vorliegenden Schnittansichten jedoch nicht alle vollständig zu sehen sind. Der Strömungsweg verläuft von dem Anschluss 2, welcher den Gaseingang bildet, zunächst zu dem Absperrventil 13 und von diesem dann durch einen nicht näher gezeigten Kanal zu dem Druckregler 16. Von dem Druckregler 16 verläuft der Strömungsweg zu dem Dosierventil 14 und von dort zu dem Anschluss 4, welcher den Ausgang bildet.

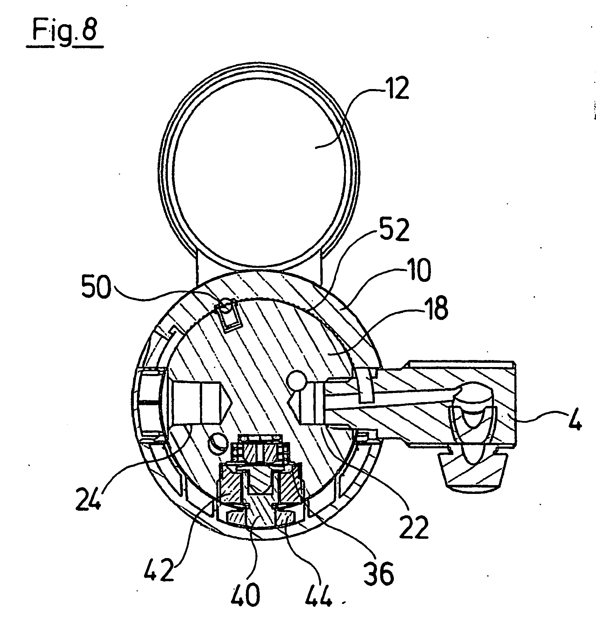

An der zweiten rückseitigen Stirnseite des Ventilblockes 18 ist ein zweiter Ausgang 20 als alternativer Anschluss zu dem Anschluss 4 ausgebildet, welcher jedoch im gezeigten Ausführungsbeispiel verschlossen ist. Dieser zweite Ausgang 20 könnte benutzt werden, wenn ein rückseitiger Ausgang gewünscht ist, beispielsweise wenn die gezeigte Gasentnahmevorrichtung fest in eine Anlage integriert werden soll. Wenn der Ausgang 20 verwendet werden soll, kann dann entsprechend auf den Anschluss 2 verzichtet werden und die entsprechende Anschlussausnehmung 22 in dem Ventilblock 18, welche in Fig. 8 zu erkennen ist, verschlossen werden. In Fig. 8 ist eine zweite Anschlussausnehmung 24 zu erkennen, welche im gezeigten Beispiel verschlossen ist. Diese bildet einen alternativen Ausgang, welcher mit den internen Strömungswegen im Ventilblock 18 so verbunden ist, dass er in Strömungsrichtung vor dem Dosierventil 14 gelegen ist. Somit würde die Verwendung der Anschlussausnehmung 24 mit einem weiteren Anschluss eine Gasentnahme mit dem vollen Fluss ohne Dosierung durch das Dosierventil 14 ermöglichen.

Das Absperrventil 13 ist als Kolbenventil mit einem axial beweglichen Kolben 26 ausgebildet. In der geschlossenen Stellung kommt der Kolben 26 an dem Ventilsitz 28 dichtend zur Anlage. Der Kolben 26 ist durch eine Druckfeder 30 in Schließrichtung vorgespannt. Das Öffnen erfolgt durch Verschwenken eines Kipphebels 32 um eine Schwenkachse, welche sich normal zu der Bewegungsachse des Kolbens 26 erstreckt. Der Kolben weist eine Nocke bzw. Exzentrizität auf, welche so gestaltet ist, dass der Kolben in der Schließstellung nicht gegen die Feder gedrückt wird. In der geöffneten Stellung drückt die Exzentrizität bzw. Nocke den Kolben gegen die Feder, so dass der Kolben von dem Ventilsitz 28 abgehoben wird und der Durchgang geöffnet wird. Das Schiebeelement 10 ist über eine Nut 34, welche in diesem ausgestaltet ist, mit dem freien Ende des Kipphebels 32 in Eingriff, so dass durch Verschieben des Schiebeelementes 10 der Kipphebel 32 um seine Schwenkachse verschwenkt wird. Die Nut 34 erstreckt sich in Umfangsrichtung um die Längsachse X, so dass bei Drehung des Schiebeelements 10 gemeinsam mit dem Drehring 8 das Ende des Kipphebels 32 in der Nut gleitet.

Das Dosierventil 14 ist als Membranventil ausgebildet, bei welchem eine Membran 36 gegenüber einem Ventilsitz 38 bewegbar ist. Die Bewegung der Membran wird dabei von einer Ventil- bzw. Gewindespindel 40 veranlasst, welche in einer Gewindeaufnahme 42 angeordnet ist. D. h. durch Drehung der Gewindespindel 40 um ihre Längsachse wird diese aufgrund des Gewindes gleichzeitig axial bewegt, wodurch die Membran auf den Ventilsitz 38 zu oder von diesem weg bewegt wird, je nach Drehrichtung. Die Gewindespindel 40 erstreckt sich über die Außenkontur des Ventilblocks 18 radial nach außen. An dem freien, sich radial nach außen erstreckenden Ende der Gewindespindel 40 ist ein Zahnrad 44, welches als Stirnrad ausgebildet ist, angeordnet. Dieses Zahnrad 44 kämmt mit einer gekrümmten Zahnstange bzw. Verzahnung 46, welche in dem Drehring 8 angeordnet ist. Zwischen dem Drehring 8 und der Zahnstange 46 ist ein Federelement 48 oder sind mehrere Federelemente 48 vorgesehen, welche die Zahnstange 46 gegen das Zahnrad 44 drücken, so dass die Verzahnungen des Zahnrades 44 und der Zahnstange 46 spielfrei in Eingriff gehalten werden. Durch Drehung des Drehringes 18 wird somit über die Zahnstange 46 das Zahnrad 44 gedreht und damit das Dosierventil 14 je nach Drehrichtung geöffnet oder geschlossen. Um eine feinfühlige Einstellung zu ermöglichen ist eine Rastung vorgesehen, welche von Kugelrastelementen 50, welche am Außenumfang des Ventilblocks 18 angeordnet sind, und einer Riffelung 52 am Innenumfang des Drehringes 8 und des Schiebeelemente 10 erzeugt wird. Es sind drei Kugelrastelemente 50 vorgesehen, welche gleichmäßig über den Außenumfang des Ventilblocks 18 verteilt sind, um eine symmetrische Krafteinleitung in den Drehring 8 zu bewirken.

Der Druckregler 16 ist in herkömmlicher Weise ausgebildet, so dass auf eine detaillierte Beschreibung verzichtet wird.

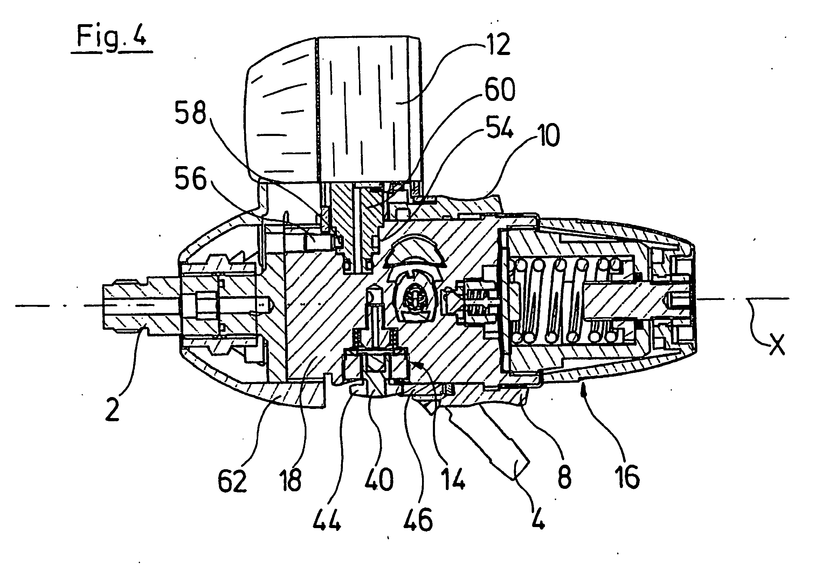

Wie in Fig. 4 zu erkennen ist, ist das Manometer 12 in einer Ausnehmung bzw. ein Sackloch 54 am Außenumfang des Ventilblockes 18 eingesteckt. Es ist dort durch eine Madenschraube 56, welche in eine Nut 58 am Anschlussvorsprung 60 des Manometers 12 eingreift, gesichert. Die Ausnehmung 54 ist mit dem ausgangsseitigen Strömungsweg des Druckreglers 16 verbunden, so dass dessen Ausgangsdruck von dem Manometer 12 angezeigt wird.

Der Ventilblock 18 ist nach außen von einem Gehäuse 62 aus Kunststoff umgeben, welches im gezeigten Beispiel aus fertigungstechnischen und montagetechnischen Gründen mehrteilig ausgebildet ist. Das Gehäuse 62 umgibt dabei nicht nur den Ventilblock, sondern auch das Manometer 12, so dass es die gesamte Gasentnahmevorrichtung nach außen abschließt und die äußere Form bzw. Gestalt der Gasentnahmevorrichtung definiert. Dabei kann das Gehäuse 62 auch nicht genutzte Öffnungen oder Anschlüsse nach außen überdecken. Dazu können in dem Gehäuse 62 entfernbare Stopfen vorgesehen sein, alternativ ist es möglich auch verschiedene Gehäuse vorzusehen, je nach dem welche Anschlüsse an dem Ventilblock 18 genutzt werden sollen. Das umgebene Gehäuse 62 aus Kunststoff ermöglicht eine ansprechende Außengestaltung der gesamten Gasentnahmevorrichtung.

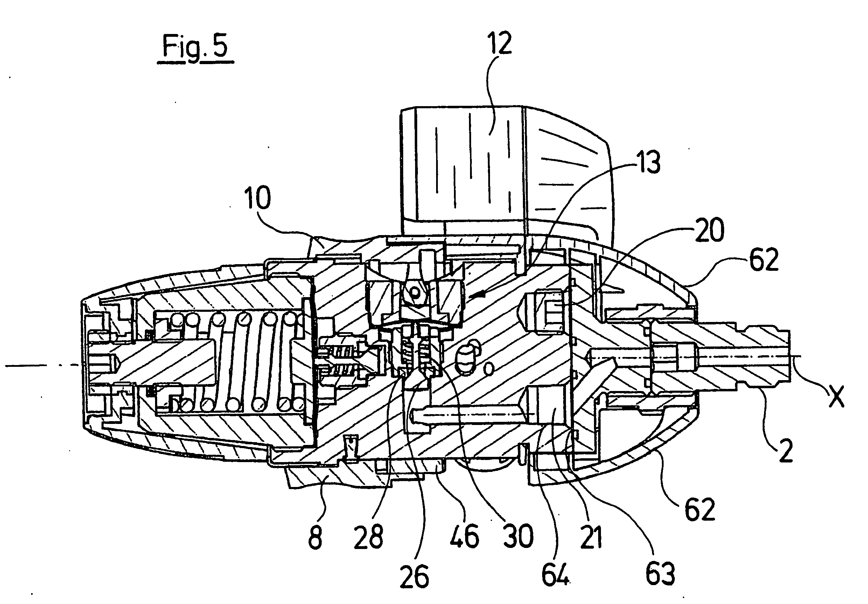

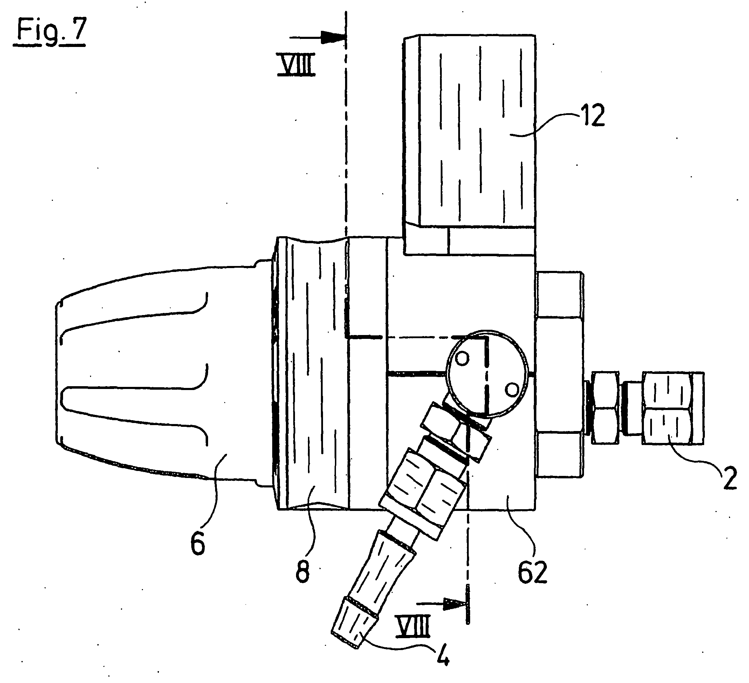

Die in dem Ventilblock 18 ausgebildeten Anschlüsse bzw. Aus- und Eingänge sind vorzugsweise so ausgebildet, dass sie sowohl zur Abdichtung mittels eines O-Ringes als auch zur Abdichtung mittels Gewinde geeignet sind. Dies wird beispielsweise anhand der Anschlussausnehmung 21 in Fig. 5 beschrieben, welche zur Verbindung mit dem Anschluss 2 als Eingang vorgesehen ist. Die Anschlussausnehmung 21 ist so ausgebildet, dass sie an ihrer offenen Außenseite zum einen einen Sitz 63 für einen O-Ring aufweist und zum anderen in ihrem Inneren mit einem Gewinde 64 versehen ist. Im gezeigten Beispiel wird das Gewinde 64 nicht genutzt und die Abdichtung erfolgt über einen O-Ring an dem Sitz 63. Es ist jedoch möglich einen Anschluss 2 direkt in das Gewinde 64 der Anschlussausnehmung 21 einzuschrauben, wie es in der Ansicht gemäß Fig. 7 dargestellt ist. Dann erfolgt die Abdichtung über das Gewinde 64.

### Bezugszeichenliste

- 2, 4: Anschluss
- 6: Handrad
- 8: Drehring
- 10: Schiebeelement
- 12: Manometer
- 13: Absperrventil
- 14: Dosierventil
- 16: Druckregler
- 18: Ventilblock
- 20: Ausgang
- 21: Anschlussausnehmung
- 22: Ausnehmung
- 24: Anschlussausnehmung
- 26: Kolben
- 28: Ventilsitz
- 30: Druckfeder
- 32: Kipphebel
- 34: Nut
- 36: Membran
- 38: Ventilsitz
- 40: Gewindespindel
- 42: Gewindeaufnahme
- 44: Zahnrad
- 46: Zahnstange
- 48: Federelement
- 50: Kugelrastelement
- 52: Riffelung
- 54: Sackloch
- 56: Madenschraube
- 58: Nut
- 60: Anschlussvorrichtung
- 62: Gehäuse
- 63: Sitz
- 64: Gewinde

- X: Längsachse

## Patentansprüche

1. Gasentnahmevorrichtung mit einem Druckregler (16) und einem Dosierventil (14), **dadurch gekennzeichnet, dass**
der Druckregler (16) und das Dosierventil (14) in einen gemeinsamen Ventilblock (18) integriert sind, in welchem Strömungskanäle zur Verbindung von Druckregler (16) und Dosierventil (14) ausgebildet sind,
der Druckregler (16) an einer ersten, in Einbaulage nach vorne weisende Stirnseite des Ventilblocks (18) angeordnet ist, an der Vorderseite ein Handrad (6) zur Betätigung des Druckreglers (18) angeordnet ist, und
das Dosierventil (14) in einer Ausnehmung an einer Umfangsfläche des Ventilblocks (18) angeordnet ist, welche sich normal zu der ersten Stirnseite erstreckt.

2. Gasentnahmevorrichtung nach Anspruch 1, **dadurch gekennzeichnet, dass** in den Ventilblock (18) zusätzlich ein Absperrventil (13) integriert ist.

3. Gasentnahmevorrichtung nach Anspruch 1 oder 2, **dadurch gekennzeichnet, dass** an den Ventilblock (18) ein Manometer (12) angesetzt ist.

4. Gasentnahmevorrichtung nach Anspruch 2 oder 3, **dadurch gekennzeichnet, dass** in dem Ventilblock (18) Verbindungskanäle zur Verbindung von Druckregler (16), Dosierventil (14) und Absperrventil (13) und/oder Manometer (12) ausgebildet sind.

5. Gasentnahmevorrichtung einem der vorangehenden Ansprüche, **dadurch gekennzeichnet, dass** der Ventilblock (18) einstückig ausgebildet ist.

6. Gasentnahmevorrichtung nach einem der Ansprüche 2 bis 5, **dadurch gekennzeichnet, dass** das Absperrventil (13) an einer Umfangsfläche des Ventilblocks (18) angeordnet sind.

7. Gasentnahmevorrichtung nach einem der vorangehenden Ansprüche, **dadurch gekennzeichnet, dass** an einer zweiten Stirnseite des Ventilblockes (18) und/oder an einer Umfangsfläche zumindest ein Gaseingang (2) und ein Gasausgang (4) angeordnet sind.

8. Gasentnahmevorrichtung nach einem der vorangehenden Ansprüche, **dadurch gekennzeichnet, dass** zur Betätigung des Dosierventils (14) ein den Ventilblock (18) umfänglich umgebender Drehring (8) angeordnet ist, welcher um die Längsachse (X) des Ventilblockes (18) drehbar ist.

9. Gasentnahmevorrichtung nach Anspruch 8, **dadurch gekennzeichnet, dass** der Drehring (8) eine Verzahnung (46) aufweist, welche mit einem Zahnrad (44) einer Ventilspindel (40) des Dosierventils (14) kämmt, wobei sich die Längsachse der Ventilspindel (40) normal zur Längsachse (X) des Ventilblockes (18) erstreckt.

10. Gasentnahmevorrichtung nach Anspruch 8 oder 9, **dadurch gekennzeichnet, dass** Drehring (8) konzentrisch zu einem Handrad (6) des Druckreglers (16) angeordnet ist.

11. Gasentnahmevorrichtung nach einem der Ansprüche 2 bis 10, **dadurch gekennzeichnet, dass** zur Betätigung des Absperrventils (13) ein Schiebeelement (10) angeordnet ist, welches parallel zur Längsachse (X) des Ventilblockes (18) bewegbar ist.

12. Gasentnahmevorrichtung nach Anspruch 11, **dadurch gekennzeichnet, dass** das Schiebelement (10) an einem Drehring (8), welcher zur Betätigung des Dosierventils vorgesehen ist, linear beweglich geführt ist.

13. Gasentnahmevorrichtung, nach einem der vorangehenden Ansprüche, **dadurch gekennzeichnet, dass** das Absperrventil (13) über eine Kipphebelmechanik (32) betätigbar ist, welche vorzugsweise über ein Schiebelement (10) betätigbar ist.

## Claims

1. A point-of-use regulator for gas, with a pressure regulator (16) and with a metering valve (14), **characterised in that**
the pressure regulator (16) and the metering valve (14) are integrated into a common valve block (18), in which flow channels are formed for connecting the pressure regulator (16) and the metering valve (14),
the pressure regulator (16) is arranged at a first end-side of the valve block (18) which is faces to the front in the installed condition, and a hand wheel (6) for actuating the pressure regulator (18) is arranged on the front side, and
the metering valve (14) is arranged in a recess on a peripheral surface of the valve block (18) which extends normally to the first end-side.

2. A point-of-use regulator for gas, according to claim 1, **characterised in that** a shut-off valve (13) is additionally integrated into the valve block (18).

3. A point-of-use regulator for gas, according to claim 1 or 2, **characterised in that** a manometer (12) is applied onto the valve block (18).

4. A point-of-use regulator for gas, according to claim 2 or 3, **characterised in that** connection channels for connecting the pressure regulator (16), the metering valve (14) and the shut-off valve (13) and/or the manometer (12), are formed in the valve block (18).

5. A point-of-use regulator for gas, according to one of the preceding claims, **characterised in that** the valve block (18) is designed as one piece.

6. A point-of-use regulator for gas, according to one of the claims 2 to 5, **characterised in that** the shut-off valve (13) is arranged on a peripheral surface of the valve block (18).

7. A point-of-use regulator for gas, according to one of the preceding claims, **characterised in that** at least one gas entry (2) and one gas exit (4) are arranged on a second end-side of the valve block (18) and/or on a peripheral surface.

8. A point-of-use regulator for gas, according to one of the preceding claims, **characterised in that** a rotary ring (8) which peripherally surrounds the valve block (18) and which is rotatable about the longitudinal axis (X) of the valve block (18), is provided for actuating the metering valve (14).

9. A point-of-use regulator for gas, according to claim 8, **characterised in that** the rotary ring (8) comprises a toothing (46), which meshes with a toothed wheel (44) of a valve spindle (40) of the metering valve (14), wherein the longitudinal axis of the valve spindle (40) extends normally to the longitudinal axis (X) of the valve block (18).

10. A point-of-use regulator for gas, according to claim 8 or 9, **characterised in that** the rotary ring (8) is arranged concentrically to a hand wheel (6) of the pressure regulator (16).

11. A point-of-use regulator for gas, according to one of the claims 2 to 10, **characterised in that** a slider element (10) which may be moved parallel to the longitudinal axis (X) of the valve block (18), is provided for actuating the shut-off valve (13).

12. A point-of-use regulator for gas, according to claim 11, **characterised in that** the slider element (10) is guided in a linearly movable manner on a rotary ring (8) which is provided for actuating the metering valve.

13. A point-of-use regulator for gas, according to one of the preceding claims, **characterised in that** the shut-off valve (13) may be actuated via rocker lever mechanics (32), which may be actuated preferably via a slider element (10).

## Revendications

1. Dispositif de prélèvement de gaz, comportant un régulateur de pression (16) et une soupape de réglage (14), **caractérisé en ce que** le régulateur de pression (18) et la soupape de réglage (14) sont intégrés dans un bloc de soupapes (18) commun, dans lequel sont configurés des canaux d'écoulement, destinés à relier le régulateur de pression (18) et la soupape de réglage (14) ;
le régulateur de pression (16) est disposé sur une première face frontale, qui en position de montage est dirigée vers l'avant, du bloc de soupapes (18), un volant (6) étant disposé sur la face avant, pour actionner le régulateur de pression (18) ; et
la soupape de réglage (14) étant disposée dans un évidement aménagé dans une surface périphérique du bloc de soupapes (18), surface qui s'étend normalement à la première face frontale.

2. Dispositif de prélèvement de gaz selon la revendication 1, **caractérisé en ce qu'**en outre une soupape d'arrêt (13) est intégrée dans le bloc de soupapes (18).

3. Dispositif de prélèvement de gaz selon la revendication 1 ou 2, **caractérisé en ce qu'**un manomètre (12) est fixé au bloc de soupapes (18).

4. Dispositif de prélèvement de gaz selon la revendication 2 ou 3, **caractérisé en ce que** des canaux de liaison sont configurés dans le bloc de soupapes (18), pour relier le régulateur de pression (16), la soupape de réglage (14) et la soupape d'arrêt (13) et/ou le manomètre (12).

5. Dispositif de prélèvement de gaz selon l'une des revendications précédentes, **caractérisé en ce que** le bloc de soupapes (18) est configuré d'une manière monobloc.

6. Dispositif de prélèvement de gaz selon l'une des revendications 2 à 5, **caractérisé en ce que** la soupape d'arrêt (13) est disposée sur une surface périphérique du bloc de soupapes (18).

7. Dispositif de prélèvement de gaz selon l'une des revendications précédentes, **caractérisé en ce qu'**au moins une entrée de gaz (2) et une sortie de gaz (4) sont disposées sur une deuxième face frontale du bloc de soupapes (18) et/ou sur une surface périphérique.

8. Dispositif de prélèvement de gaz selon l'une des revendications précédentes, **caractérisé en ce qu'**une bague tournante (8), entourant la périphérie du bloc de soupapes (18), et qui peut tourner autour de l'axe longitudinal (X) du bloc de soupapes (18), est disposée pour actionner la soupape de réglage (14).

9. Dispositif de prélèvement de gaz selon la revendication 8, **caractérisé en ce que** la bague tournante (8) présente un engrenage (46), qui entre en prise avec une roue dentée (44) d'une tige de soupape (40) de la soupape de réglage (14), l'axe longitudinal de la tige de soupape (40) s'étendant normalement par rapport à l'axe longitudinal (X) du bloc de soupapes (18).

10. Dispositif de prélèvement de gaz selon la revendication 8 ou 9, **caractérisé en ce que** la bague tournante (8) est disposée d'une manière concentrique à un volant (6) du régulateur de pression (16).

11. Dispositif de prélèvement de gaz selon l'une des revendications 2 à 10, **caractérisé en ce qu'**un élément coulissant (10), qui est mobile parallèlement à l'axe longitudinal (X) du bloc de soupapes (18), est disposé pour actionner la soupape d'arrêt (13).

12. Dispositif de prélèvement de gaz selon la revendication 11, **caractérisé en ce que** l'élément coulissant (10) est guidé en pouvant subir un déplacement linéaire contre une bague tournante (8), qui est prévue pour actionner la soupape de réglage.

13. Dispositif de prélèvement de gaz selon l'une des revendications précédentes, **caractérisé en ce que** la soupape d'arrêt (13) peut être actionnée par l'intermédiaire d'un mécanisme à culbuteurs (32), qui, de préférence, peut être actionné par l'intermédiaire d'un élément coulissant (10).
